# FASCICULE DE BREVET EUROPEEN

(11) **EP 0 925 778 B1**
(45) Date de publication et mention de la délivrance du brevet: **18.07.2001**
(21) Numéro de dépôt: 98402900.9
(22) Date de dépôt: 20.11.1998
(51) Int. Cl.: A61K 7/48

(54) **Composition cosmétique ou dermatologique comprenant un polymère filmogène en dispersion et une emulsion aqueuse de silicone**
Kosmetische oder dermatologische Zusammensetzung enthaltend ein dispergiertes filmbildendes Polymer und eine wässrige Dispersion eines Silikons
Cosmetic or dermatological composition comprising a dispersed filmforming polymer and an aqueous dispersion of a silicone

(30) Priorité: 05.12.1997 FR 9715414
(43) Date de publication de la demande: 30.06.1999
(73) Titulaire: L'OREAL, 75008 Paris (FR)
(72) Inventeur: De la Poterie, Valérie, 77820 Le Chatelet en Brie (FR); Bara, Isabelle, 75013 Paris (FR); Mellul, Myriam, 94240 L'Hay les Roses (FR)
(74) Mandataire: Lhoste, Catherine

(56) Documents cités:
- EP-A- 0 775 483
- EP-A- 0 793 957
- US-A- 5 389 363

## Description

La présente invention a trait à une composition cosmétique ou dermatologique susceptible d'être appliquée sur la peau et/ou les lèvres. Cette composition comprend en particulier un polymère filmogène, sous forme de dispersion aqueuse de particules, et peut être utilisée en tant que produit de maquillage.

Les compositions à appliquer sur la peau, et/ou les lèvres telles que les rouges à lèvres et les fonds de teint, se présentent généralement sous forme de stick, de pâte souple ou de pâte coulée, et comprennent des corps gras tels que des huiles, des composés pâteux et/ou des cires, et une phase particulaire généralement composée de charges et de pigments. Ces compositions, lorsqu'elles sont appliquées sur la peau ou les lèvres, présentent toutefois l'inconvénient de transférer. On entend par là que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support. Il s'en suit donc une persistance médiocre de la composition sur la peau, les semi-muqueuses ou les muqueuses, et la nécessité de renouveler régulièrement son application. Par ailleurs, l'apparition de traces inacceptables sur certains vêtements et notamment sur les cols de chemisier peut écarter certaines femmes de l'utilisation de ce type de maquillage.

Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, produites par les mouvements des paupières. On a encore constaté que les eye-liners pouvaient également couler. Tous ces phénomènes engendrent un effet inesthétique que l'on souhaite bien évidemment éviter.

Depuis plusieurs années, de nombreux cosméticiens se sont intéressés aux compositions cosmétiques, notamment de rouge à lèvres ou de fond de teint 'sans transfert'. Ainsi, il a été envisagé, dans JP-A-61/65809, des compositions liquides de rouge à lèvres 'sans transfert' contenant de 1 à 70% en poids de résine de silicone à motifs répétitifs silicates, de 10 à 98% en poids d'une huile de silicone volatile et des charges pulvérulentes. Toutefois, le film obtenu sur les lèvres après évaporation de l'huile de silicone présente l'inconvénient de devenir inconfortable au cours du temps (sensation de dessèchement et de tiraillement).
On connaît également, par EP-A-602905, des rouges à lèvres 'sans transfert' contenant une silicone volatile et une résine de silicone liquide comportant une chaîne estérifiée pendante ayant au moins 12 atomes de carbone. Le film de rouge à lèvres déposé présente notamment l'inconvénient de manquer de confort à l'application, en particulier d'être trop sec.

Ainsi, d'une manière générale, l'association d'huiles volatiles avec certains composés siliconés permet d'améliorer les propriétés de 'sans transfert'. Toutefois, les films obtenus après application de ces compositions et évaporation des volatils présentent néanmoins l'inconvénient d'être relativement mats, et conduisent ainsi à un maquillage peu brillant. En outre, les propriétés de sans transfert ne sont pas totales.

D'autre part, il a été décrit des compositions cosmétiques de maquillage comprenant des polymères filmogènes en solution aqueuse; mais ces compositions sont sensibles à l'eau et ne peuvent donc pas, notamment, être appliquées sur les lèvres. Par ailleurs, lorsque les polymères sont solubilisés dans des milieux alcooliques ou hydroalcooliques, on a constaté que la composition obtenue pouvait engendrer des problèmes d'irritation et/ou de déshydratation de la peau, d'où un inconfort certain pour l'utilisatrice.

Il subsistait donc le besoin d'une composition cosmétique qui transférait peu ou pas du tout, c'est-à-dire d'une composition 'sans transfert', tout en possédant de bonnes propriétés cosmétiques.

Il a alors été proposé dans les documents EP-A-775483 et EP-A-793957, une composition cosmétique comprenant, dans un système polymérique, une dispersion aqueuse de particules de polymère filmogène. Ce système permet l'obtention d'un film cohésif sur le support sur lequel il est déposé, film qui résiste remarquablement bien au transfert et présente une bonne résistance à l'eau.

Toutefois, on a constaté que, selon la nature du polymère employé, le démaquillage à l'aide de certains démaquillants usuels était peu aisé, notamment lorsque l'on utilisait des démaquillants huileux, du fait de la bonne résistance chimique desdits polymères à l'eau et/ou aux corps gras. En outre, dans certains cas, le film de polymère peut être éliminé à l'aide de solvants hydrocarbonés, dont l'utilisation en cosmétique n'est pas souhaitable. Dans d'autres cas, il est nécessaire d'utiliser des démaquillants spécifiques, ce qui est contraignant pour l'utilisateur. D'autre part, on a également constaté que le film déposé sur le support pouvait être légèrement collant, même après séchage.

Il est par ailleurs connu du document US-A-5,389,363 des compositions de mascara contenant des gels de polymères dispersibles dans l'eau et des silicones. La quantité de polymère dispersible enseignée est trop faible pour former un film cohésif sur les lèvres ou la peau, résistant aux mouvements des lèvres et/ou de la peau.

La présente invention a justement pour but de proposer une composition cosmétique ou dermatologique qui permet d'obtenir un film de très bonne tenue, qui ne transfère pas et ne tache pas un support avec lequel il serait en contact, et qui ne migre pas au cours du temps, tout en possédant de bonnes propriétés cosmétiques ou dermatologiques. De plus, ledit film peut être très peu, voire pas du tout, collant, notamment sur les lèvres, et est de surcroît aisément démaquillable à l'aide des produits de démaquillage existants, qu'ils soient aqueux, hydroalcooliques ou huileux.

Un objet de l'invention est une composition cosmétique ou dermatologique susceptible d'être appliquée sur la peau et/ou les lèvres, telle que définie dans la revendication 1.

Un autre objet est une composition de maquillage ou de soin sans transfert, telle que définie dans la revendication 19.

Un autre objet est une composition de maquillage des lèvres du visage et/ou de la peau des êtres humains, telle que définie dans la revendication 20.

Un autre objet est l'utilisation de l'association d'un système polymérique en quantité efficace qui comprend des particules d'au moins un polymère filmogène en dispersion dans un milieu cosmétiquement acceptable et d'une émulsion aqueuse d'au moins un composé siliconé, dans une composition cosmétique susceptible d'être appliquée sur la peau et/ou les lèvres, afin d'obtenir sur celles-ci un film cohésif de très bonne tenue et/ou qui ne transfère pas et/ou qui ne migre pas et/ou qui ne tache pas et/ou qui ne colle pas et/ou brillant.

Un autre objet est l'utilisation de l'association d'un système polymérique en quantité efficace qui comprend des particules d'au moins un polymère filmogène en dispersion dans un milieu cosmétiquement acceptable, et d'une émulsion aqueuse d'au moins un composé siliconé, dans une composition cosmétique susceptible d'être appliquée sur la peau et/ou les lèvres, pour diminuer le transfert et/ou la migration de ladite composition.

Un autre objet est l'utilisation de l'association d'un système polymérique en quantité efficace qui comprend des particules d'au moins un polymère filmogène en dispersion dans un milieu dermatologiquement acceptable, et d'une émulsion aqueuse d'au moins un composé siliconé, pour la fabrication d'une composition destinée à traiter thérapeutiquement la peau et/ou les lèvres et former sur celles-ci un film cohésif suivant leurs mouvements.

La composition selon l'invention permet l'obtention d'un film cohésif homogène, qui présente une texture légère et reste confortable à porter tout au long de la journée. Le film est mou, souple, élastique et flexible sur la peau; il suit les mouvements du support sur lequel il est déposé, sans se craqueler et/ou se décoller. Il adhère notamment parfaitement sur les lèvres du visage. D'autre part, le film obtenu peut être très brillant, ou plus ou moins mat, selon la nature des constituants de la composition, d'où une gamme plus étendue de produits de maquillage, brillants ou mats, au choix, par rapport à l'art antérieur.

Un autre avantage apporté par la présente invention est qu'il est possible d'obtenir un film sans transfert très doux et très lisse, non collant, sans effet 'gras', très résistant à l'eau et très confortable, en particulier pour formuler des rouges à lèvres, des fonds de teint, des eye-liner ou des fards à paupières.

La composition selon l'invention est facilement applicable et s'étale aisément en particulier sur les lèvres du visage. La composition selon l'invention trouve notamment une application particulièrement intéressante dans le domaine du soin et/ou du maquillage de la peau et/ou les lèvres des êtres humains. Ainsi, la composition selon l'invention trouve une application préférée dans le domaine des produits de maquillage des lèvres du visage, notamment en tant que rouge à lèvres. Elle trouve également une autre application avantageuse dans le domaine des eye-liners, des fonds de teint ou des fards à joues ou à paupières. Elle présente, en outre, l'avantage de se démaquiller avec les démaquillants classiques contrairement aux compositions de l'art antérieur.

La composition selon l'invention comprend donc un système polymérique qui comprend au moins un polymère filmogène sous forme dispersé, c'est-à-dire sous forme de dispersion de particules, notamment dans un milieu cosmétiquement ou dermatologiquement acceptable. De préférence, le polymère filmogène se présente sous forme de dispersion aqueuse de particules dudit polymère.

La dispersion aqueuse comprenant un ou plusieurs polymères filmogènes peut être préparée par l'homme du métier sur base de ses connaissances générales, notamment par polymérisation en émulsion ou par mise en dispersion du polymère préalablement formé.

Parmi les polymères filmogènes utilisables dans la composition selon la présente invention, on peut citer les polymères synthétiques, de type polycondensat ou de type radicalaire, les polymères d'origine naturelle, et leurs mélanges.

On peut ainsi citer, parmi les polycondensats, les polyuréthannes anioniques, cationiques, non ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes, et leurs mélanges. Le polyuréthanne peut être, par exemple, un copolymère polyuréthanne, polyurée/uréthanne ou polyurée, aliphatique, cycloaliphatique ou aromatique, comportant, seule ou en mélange,
. au moins une séquence d'origine polyester aliphatique linéaire ou ramifié et/ou cycloaliphatique et/ou aromatique, et/ou
. au moins une séquence d'origine polyéther aliphatique et/ou cycloaliphatique et/ou aromatique, et/ou
. au moins une séquence siliconée, substituée ou non, ramifiée ou non, par exemple polydiméthylsiloxane ou polyméthylphénylsiloxane, et/ou
. au moins une séquence comportant des groupes fluorés.
Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

On peut également citer les polyesters, les polyesters amides, les polyesters à chaîne grasse, les polyamides, et les résines époxyesters.

Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylène diamine, l'hexaméthylènediamine, la méta- ou para-phénylène diamine. Comme aminoalcool, on peut utiliser la monoéthanolamine.

Comme monomère porteur de groupement anionique pouvant être utilisé lors de la polycondensation, on peut citer par exemple l'acide diméthylol propionique, l'acide trimellitique ou un dérivé tel que l'anhydride trimellitique, le sel de sodium de l'acide sulfo-3 pentanediol, le sel de sodium de l'acide 5-sulfo 1,3-benzènedicarboxylique.
Les polyesters à chaîne grasse peuvent être obtenus par l'utilisation de diols à chaîne grasse lors de la polycondensation. Les résines époxyesters peuvent être obtenues par polycondensation d'acides gras avec un condensât aux extrémités α, ω - diépoxy.

Les polymères de type radicalaires peuvent être notamment des polymères, ou des copolymères, acryliques et/ou vinyliques. On utilise de préférence des polymères radicalaires anioniques. Comme monomère porteur de groupement anionique pouvant être utilisé lors de la polymérisation radicalaire, on peut citer l'acide acrylique, l'acide méthacrylique, l'acide crotonique, l'anhydride maléique, l'acide acrylamido-2 méthyl-2 propane sulfonique.
Les polymères acryliques peuvent résulter de la copolymérisation de monomères choisis parmi les esters et/ou les amides de l'acide acrylique ou de l'acide méthacrylique. Comme exemple de monomères de type ester, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle. Comme exemple de monomères de type amide, on peut citer le N-t-butyl acrylamide et le N-t-octyl acrylamide.
On utilise de préférence des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxypropyle, le méthacrylate d'hydroxyéthyle, le méthacrylate de 2-hydroxypropyle.
Les polymères vinyliques peuvent résulter de l'homopolymérisation ou de la copolymérisation de monomères choisis parmi les esters vinyliques, le styrène ou le butadiène. Comme exemple d'esters vinyliques, on peut citer l'acétate de vinyle, le néodécanoate de vinyle, le pivalate de vinyle, le benzoate de vinyle et le t-butyl benzoate de vinyle. On peut également utiliser des copolymères acryliques/silicones, ou encore des copolymères nitrocellulose/acryliques.

Les polymères d'origine naturelle, éventuellement modifiés, peuvent être choisis parmi la résine shellac, la gomme de sandaraque, les dammars, les élémis, les copals, les dérivés cellulosiques, et leurs mélanges.
On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La composition selon l'invention peut, en outre, contenir des polymères hydrosolubies comme les dérivés de protéines d'origine animale ou végétale et plus particulièrement les hydrolysats de kératine et les kératines sulfoniques, la polyvinylpyrrolidone, les copolymères vinyliques tels que le copolymère de l'éther méthyivinylique et de l'anhydride maléique ou le copolymère de l'acétate de vinyle et de l'acide crotonique, les glycoaminoglycanes, l'acide hyaluronique et ses dérivés, les polymères acryliques, les polysaccharides, les polymères cellulosiques et leurs dérivés. Ces polymères seront en particulier utilisés si l'on souhaite une élimination plus ou moins sensible du film, à l'eau.

Afin d'améliorer le caractère filmogène d'un polymère, par exemple en abaissant sa température de transition vitreuse, il est possible d'ajouter au système polymérique un agent de coalescence, qui peut être choisi parmi les agents de coalescence connus.

Lorsque l'on emploie une dispersion aqueuse de particules de polymère, la teneur en matière sèche de ladite dispersion aqueuse peut être de l'ordre de 5-60% en poids, et de préférence 30-50%. La taille des particules de polymères en dispersion aqueuse peut être comprise entre 10-500 nm, et est de préférence comprise entre 20 et 150 nm, ce qui permet d'obtenir un film ayant une brillance remarquable.

La composition peut comprendre de 2-60% en poids, mieux de 5 à 60 %, de préférence 2-30% en poids de matière sèche de polymères filmogènes. De façon plus générale, la quantité totale de polymère, doit être en quantité suffisante pour former sur la peau et/ou sur les lèvres un film cohésif, susceptible de suivre les mouvements de la peau et/ou des lèvres sans se décoller, ni craqueler.

La composition peut également comprendre au moins un agent plastifiant, hydrophile ou hydrophobe, choisi pour sa compatibilité avec le ou les polymères et en quantité telle qu'elle ne nuit pas à la sensibilité du film à l'eau. Ledit agent plastifiant peut être choisi parmi tous les composés connus de l'homme du métier comme étant susceptibles de remplir la fonction recherchée. Cet agent peut être hydrosoluble ou insoluble dans l'eau et peut éventuellement se présenter sous forme de dispersion aqueuse.
En particulier, on peut citer, seuls ou en mélange, les plastifiants usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther,
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,
La quantité d'agent plastifiant peut être choisie par l'homme du métier sur base de ses connaissances générales, de manière à obtenir un film ayant les propriétés mécaniques souhaitées, tout en conservant à la composition des propriétés cosmétiquement acceptables.

Selon l'invention, on appellera 'système polymérique' le mélange comprenant le ou les polymères filmogènes, les éventuels agents de coalescence et les éventuels plastifiants; ce système polymérique doit être apte à former un film sur le support sur lequel il est déposé, souple, flexible, cohésif, suivant les mouvements du support (lèvres ou peau) sur lequel il est disposé.
De préférence, ledit film vérifiera, dans les conditions de mesure définies avant les exemples, au moins une des conditions physico-chimiques suivantes :
. un module de Young inférieur à environ 200 MPa, de préférence inférieur à environ 100 MPa, et préférentiellement inférieur à 80 MPa, et/ou
. une élongation supérieure à environ 200%, et, de manière préférentielle, supérieure à 300%, et/ou
. une dureté inférieure à 110, de préférence inférieure à 70, plus préférentiellement inférieure à 55.

La composition selon l'invention comprend, par ailleurs, une émulsion aqueuse d'au moins un composé siliconé.
Par émulsion aqueuse, on entend une émulsion de type huile-dans-eau dans laquelle l'eau est la phase continue et la silicone est la phase dispersée. Cette émulsion peut être stabilisée par un système émulsionnant usuel.
Ladite émulsion, qui peut avoir une taille des gouttelettes de silicone inférieure à 1 µm, peut également se présenter sous forme de microémulsion transparente, ayant une taille de gouttelettes de l'ordre de 10 à 80 nm. Les microémulsions de silicone sont des émulsions stables de particules colloïdales.

Les composés siliconés présents dans la phase dispersée de l'émulsion sont de préférence des polyorganosiloxanes, qui peuvent se présenter sous forme d'huiles, de gommes, de résines ou de cires.

Lesdits composés siliconés sont généralement facilement émulsifiables dans un milieu aqueux.

Les gommes, cires et résines de silicone peuvent être ajoutées à la composition sous la forme d'émulsion dans l'eau, ou en mélange avec des silicones liquides dans lesquelles elles sont solubilisées.
D'une façon générale, les composés siliconés sont de préférence des polymères contenant des motifs répétitifs de formule : RₙSiO_{(4-n)/2}

Les substituants R présents dans ces motifs répétitifs sont des groupements organiques, et peuvent être identiques ou différents. En outre, un même composé peut contenir des motifs répétitifs différents.

Les motifs répétitifs correspondant à n = confèrent généralement au composé une structure linéaire ou cyclique dont la chaîne est constituée de liaisons siloxane. Dans le cas d'un polymère linéaire, des motifs correspondant à n = 3 constituent les groupements terminaux.
En outre, les polyorganosiloxanes peuvent contenir des motifs réticulants intercalés entre les motifs répétitifs. Ces motifs réticulants correspondent à la formule ci-dessus avec n = 1 ou n = 0.

Dans les motifs répétitifs (n = 2) et les motifs réticulants correspondant à n = 1, les groupements R peuvent représenter notamment des groupements alkyle, cycloalkyle ou aryle et peuvent comporter en outre, des groupements fonctionnels tels qu'éthers, amines, carboxyles, hydroxyles, thiols, esters, sulfonates, sulfates.
Les groupements alkyle ont par exemple 1 à 20 atomes de carbone; les groupements cycloalkyle ont par exemple 5 ou 6 chaînons; les groupements aryle sont notamment des groupements phényle.
Dans le cas des groupements terminaux correspondant à n = 3, l'un des groupements R attachés au silicium terminal peut en outre représenter un autre groupement, tel qu'un groupement OH.

Parmi les composés siliconés susceptibles d'être utilisés dans la présente invention, on peut citer les silicones non volatiles, choisies en particulier parmi les polyorganosiloxanes et notamment les polyalkylsiloxanes, les polyarylsiloxanes, les polyalkylarylsiloxanes, les copolymères polyéthersiloxanes, organomodifiés ou non, les gommes et résines de silicones, les polysiloxanes modifiés par des groupements organofonctionnels ainsi que leurs mélanges.

Les composés siliconés sont choisis plus particulièrement parmi les polyalkylsiloxanes, parmi lesquels on peut citer principalement les polydiméthylsiloxanes linéaires à groupements terminaux triméthylsilyle ayant une viscosité de 5.10° à 2,5 m²/s à 25°C, de préférence de 1.10⁻⁵ à 1 m²/s à 25°C, et notamment les produits commerciaux suivants:
- les huiles SILBIONE des séries 47 et 70047 commercialisées par RHONE POULENC telles que par exemple l'huile 47V500000,
- les huiles de la série 200 de DOW CORNING,
- les huiles VISCASIL de GENERAL ELECTRIC,
- certaines huiles des séries SF (SF96, SF18) de GENERAL ELECTRIC.

On peut également citer les polydiméthylsiloxanes linéaires à groupements terminaux diméthylsilanol tels que les huiles de la série 48 de RHONE POULENC.

On peut également citer les produits vendus sous les dénominations ABIL WAX 9800 et 9801 par la société GOLDSCHMIDT qui sont des polyalkyl (C₁-C₂₀)-siloxanes.

Parmi les polyalkylarylsiloxanes, on peut citer les polydiméthylméthylphénylsiloxanes et les polydiméthyldiphénylsiloxanes, linéaires ou ramifiés, ayant une viscosité de 1.10⁻⁵ à 5.10⁻² m²/s à 25°C, et notamment les produits commerciaux suivants :
- les huiles SILBIONE de la série 70641 de RHONE POULENC,
- les huiles des séries RHODORSIL 70633 et 763 de RHONE POULENC,
- l'huile DC 556 Fluid de DOW CORNING,
- les silicones de la série PK de BAYER comme-le produit PK20,
- les silicones des séries PN et PH de BAYER comme les produits PN1000 et PH1000,
- certaines huiles des séries SF de GENERAL ELECTRIC telles que SF1023, SF1154, SF1250 et SF1265.

Les gommes de silicone utilisables conformément à la présente invention, sont notamment des polydiorganosiloxanes ayant des masses moléculaires élevées, de préférence comprises entre 200.000 et 1.000.000. Elles peuvent être utilisées seules ou en mélange dans un solvant qui peut être choisi parmi les silicones volatils, les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.
On peut citer plus particulièrement les produits suivants : les gommes polydiméthylsiloxane/méthylvinylsiloxane, polydiméthylsiloxane/diphénylsiloxane, polydiméthylsiloxane/phénylméthylsiloxane, polydiméthylsiloxane/diphénylsiloxane/ méthylvinylsiloxane.
On peut notamment employer des mélanges tels que :
- les mélanges formés à partir d'un polydiméthylsiloxane hydroxylé en bout de chaîne (diméthiconol selon le CTFA) et d'un polydiméthylsiloxane cyclique (cyclométhicone selon le CTFA) tel que le produit Q2-1401 vendu par la société DOW CORNING,
- les mélanges formés à partir d'une gomme polydiméthylsiloxane avec une huile de silicone cyclique tel que le produit SF1214 de GENERAL ELECTRIC (qui est un mélange de gomme diméthicone ayant un poids moléculaire de 500.000 solubilisée dans le décaméthylcyclopentasiloxane).
- les mélanges de deux PDMS de viscosités différentes, notamment d'une gomme PDMS et d'une huile PDMS, tels que le produit SF1236 de la société GENERAL ELECTRIC (qui est un mélange de 15% de gomme diméthicone ayant un poids moléculaire de 500.000, d'une viscosité de 20m²/s et de 85% d'huile SF96 d'une viscosité de 5.10⁻⁶ m²/s).

Les résines d'organopolysiloxanes utilisables selon l'invention sont notamment des systèmes siloxaniques réticulés renfermant les unités R₂SiO, RSiO_{3/2} et SiO₂. Parmi ces composés, les produits plus particulièrement préférés sont ceux dans lesquels R désigne un alkyle en C₁-C₆ ou phényle.
Parmi ces résines, on peut citer le produit vendu sous la dénomination DOW CORNING 593 ou ceux vendus sous les dénominations Silicone Fluid SS4230 et SS4267 par la société GENERAL ELECTRIC et qui sont de type diméthyl/triméthyl siloxane.

Les silicones organomodifiées sont des composés siliconés tels que définis ci-dessus, comportant en outre dans leur structure un ou plusieurs groupements organofonctionnels directement fixés sur la chaîne siloxanique ou fixés par l'intermédiaire d'un radical hydrocarboné.
On peut citer, par exemple, les silicones comportant :
- des groupements polyéthylèneoxy et/ou polypropylèneoxy comportant éventuellement des groupes alkyles tels que :
- les diméthicone copolyols et notamment celui vendu par la société DOW CORNING sous la dénomination DC1248,
- les alkylméthicone copolyols et notamment l'alkyl (C₁₂) méthicone copolyol vendu par la société DOW CORNING sous la dénomination Q2-5200.
- les huiles SILWET L 722, L 7500, L 77, L 711 de la société UNION CARBIDE.
- des groupements aminés, éventuellement substitués, comme les produits vendus sous la dénomination GP4 Silicone Fluid et GP7100 par la société GENESEE ou les produits vendus sous les dénominations Q2-8220 et DC929 par la société DOW CORNING. Les groupements aminés substitués sont en particulier des groupements aminoalkyle en C₁-C₄.
- des groupements thiols comme dans les GP 72 A et GP 71 de GENESEE.
- des groupements carboxylates comme dans les produits décrits dans le brevet EP 186 507 de la société CHISSO CORPORATION.
- des groupements alkoxylés, comme les produits vendus sous la dénomination Silicone copolymer F-755 par SWS SILICONES, ou ABIL WAX 2428, 2434 et 2440 par la société GOLDSCHMIDT.
- des groupements hydroxylés, comme les polyorganosiloxanes à fonction hydroxyalkyle notamment décrits dans la demande de brevet français 85FR-163 34.
- des groupements acyloxyalkyle, comme par exemple les polyorganosiloxanes décrits dans la demande de brevet français 88FR-17433. Ces composés peuvent être préparés par estérification de polyorganosiloxanes à fonction hydroxyalkyle.
- des groupements anioniques de type carboxylique tels que les groupements alkylcarboxyliques; 2-hydroxy alkylsulfonate ou 2-hydroxyalkylthiosulfate,
- des groupements fluorés.

Parmi les composés siliconés susceptibles d'être utilisés dans la présente invention, on peut encore citer les silicones volatiles (à température ambiante et à pression atmosphérique), qui possèdent généralement un point d'ébullition compris entre 60 et 260°C et une viscosité <0.08 cm²/s (8 cSt), et en particulier :
- les silicones cycliques comportant de 3 à 7 atomes de silicium et de préférence 4 à 6 atomes de silicium tels que l'octaméthylcyclotétrasiloxane, le décaméthylcyclopentasiloxane, le dodécaméthylcyclohexasiloxane et leurs mélanges.
   On peut également citer les cyclocopolymères tels que le diméthylsiloxane/méthylalkylsiloxane, et notamment la silicone volatile FZ 3109 vendue par la société UNION CARBIDE.
   On peut encore citer les mélanges de silicones cycliques avec des composés dérivés du silicium tels que le mélange d'octaméthylcyclotétrasiloxane et de tétra triméthylsilyl pentaérythritol (50/50) et le mélange d'octaméthylcyclotétrasiloxane et d'oxy-1,1'(hexa-2,2,2',2',3,3' triméthylsilyloxy) bis-néopentane.
- les silicones volatils linéaires ayant 2 à 9 atomes de carbone et une viscosité inférieure ou égale à 5.10⁻⁶ m²/s à 25°C, tels que l'hexaméthyldisiloxane ou le décaméthyltétrasiloxane. Des silicones entrant dans cette classe sont également décrits dans l'article publié dans Cosmetic and Toiletries, Vol 91, Jan 76, p 27-32 intitulé "Volatile Silicone fluids for cosmetics".

L'émulsion aqueuse de composés siliconés pourra aisément être préparée par l'homme du métier, sur base de ses connaissances générales.

Parmi les émulsions aqueuses de silicone commercialement disponibles, on peut citer :
- les émulsions de polydiméthylsiloxane (SM2162 de General Electric),
- les émulsions de stéaryldiméthicone (SLM23032 de Wacker),
- les microémulsions d'amodiméthicone (Microémulsion 71827 de Rhône-Poulenc),
- les microémulsions cationiques de polydiméthylsiloxane à groupements aminoéthylaminopropyl (DC939 de Dow Corning),
- les microémulsions de polydiméthylsiloxane (SILTECH MFF 5015-70 de Siltech ou DC2-1281 de Dow Corning).

Les composés siliconés peuvent être présents dans la composition à raison de 0,1% à 30% en poids de matière sèche de silicone, de préférence à raison de 5% à 30% en poids.

Par ailleurs, dans un mode préféré de réalisation de l'invention, la composition peut comprendre une dispersion aqueuse de cire, de préférence une microdispersion aqueuse de cire. On peut également incorporer les cires sous forme particulaire dispersées dans l'eau.
On entend par microdispersion de cire, une dispersion aqueuse de particules de cire, dans laquelle la taille desdites particules de cire est inférieure ou égale à environ 1 micron.
Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une émulsion du type huile-dans-eau. Au refroidissement, on obtient une microdispersion stable de particules colloïdales solides de cire.

Les particules de la microdispersion de cire ont de préférence des dimensions moyennes inférieures à 1µm, de préférence inférieures à 0,5µm.
Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Les cires susceptibles d'être utilisées dans les compositions selon l'invention sont choisies parmi les cires, solides et rigides à température ambiante, d'origine animale, végétale, minérale ou de synthèse et leurs mélanges. De préférence, les cires entrant dans la composition peuvent présenter un point de fusion supérieur à 45°C environ, et en particulier supérieur à 55°C, et/ou un indice de pénétration de l'aiguille à 25°C de préférence compris entre 3 et 40, mesuré selon la norme américaine ASTM D 5 ou selon la norme française NFT 004.

On peut notamment citer la cire d'abeilles, la cire de lanoline, et les cires d'insectes de Chine, la cire de riz, la cire de Carnauba, la cire de Candellila, la cire d'Ouricurry, la cire d'Alfa, la cire de fibres de liège, la cire de canne à sucre, la cire du Japon, la cire de sumac, la cire de montan, les cires microcristallines, les paraffines, l'ozokérite, les cires de polyéthylène, les cires obtenues par la synthèse de Fisher-Tropsch et les copolymères cireux ainsi que leurs esters.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.
On peut encore citer les cires de silicone.

Il est également possible d'utiliser des mélanges commerciaux de cires autoémulsionnables contenant une cire et des tensioactifs. On peut utiliser par exemple la cire commercialisée sous la dénomination 'Cire Auto Lustrante OFR' par Tiscco, qui contient des cires de Carnauba et de paraffine en association avec des tensioactifs non ioniques, ou la cire auto-émulsionnable commercialisée sous la dénomination 'Cerax A.O. 28/B' par La Ceresine, qui contient de la cire d'Alfa en association avec un tensioactif non ionique. Ces mélanges commerciaux permettent de préparer des microdispersions de cires par simple addition d'eau.
On peut encore citer les produits 'Aquacer' de Byk Cera, et notamment : le mélange de cires synthétiques et naturelles avec émulsionnant anionique (Aquacer 520), la cire de polyéthylène avec émulsionnant non ionique ( Aquacer 514 ou 513), la cire polymérique avec émulsionnant anionique (Aquacer 511). On peut également citer le mélange de cires de polyéthylène et de paraffine avec émulsionnant non ionique ' Jonwax 120' de Johnson Polymer.

De préférence, au moins une des cires est fluorée.
Par cire fluorée, on entend une cire dont la structure chimique comprend au moins un groupement fluoré ou perfluoré, ou un mélange de cires dont au moins une comprend un groupement fluoré, ou perfluoré.
On peut citer les mélanges micronisés de cire de polyéthylène et de cire de PTFE (polytétrafluoroéthylène).
Parmi les microdispersions de cires fluorées commerciales utilisables selon l'invention, on peut citer la 'Microdispersion 411' de MicroPowders, qui est un mélange de cires de polyéthylène, de polytétrafluoroéthylène et de polymère acrylique. On peut citer, comme cire fluorée, le produit 'Aquapolyfluo 411' de Micro-Powders, qui est un mélange de cire de polyéthylène et de polytétrafluoroéthylène.

La composition selon l'invention peut comprendre 0 à 50% de matière sèche de cire, plus particulièrement 0,1-30% en poids. La (micro-)dispersion aqueuse comprend de préférence entre 10 et 70% en poids de matière sèche de cire.

La composition selon l'invention peut comprendre une quantité suffisante de tensioactif pour permettre d'obtenir une microdispersion de cire et/ou une émulsion de silicone, ainsi qu'une composition finale, stable. Notamment, elle peut comprendre 0.01 à 30% en poids de tensioactif usuel, pouvant être choisi parmi les composés suivants :
- les tensioactifs anioniques, notamment des sels d'acides gras éventuellement insaturés, ayant par exemple 12 à 18 atomes de carbone; des sels alcalins ou sels de bases organiques des acides alkyl-sulfuriques et alkylsulfoniques ayant 12 à 18 atomes de carbone ou d'acides alkyl-arylsulfoniques dont la chaîne alkyle contient 6-18 atomes de carbone; les éthers-sulfates.
- les tensioactifs non ioniques, notamment des tensioactifs polyalcoxylés et/ou polyglycérolés, et en particulier des acides gras ou amides d'acide gras; des alcools gras ou des alkylphénols; les esters d'acides gras et de polyols; les aicanediols et les alkyléthers d'alcanediols. On peut citer également les alkylcarbamates de triglycérol, les dérivés oxyéthylénés ou propoxylés des alcools de lanoline, des acides gras de la lanoline, ou de leur mélanges.
- les tensioactifs cationiques, notamment les dérivés d'ammonium quaternaire.

La cire ou mélange de cires, peut être associé à un ou plusieurs additifs gras (huileux et/ou pâteux). On peut notamment citer les huiles végétales comme l'huile de tournesol, l'huile de jojoba; les huiles minérales comme l'huile de paraffine; les huiles de silicones: la vaseline, la lanoline; les huiles fluorées; les huiles hydrocarbonées à groupement perfluoré; les esters d'alcools gras.

Il est possible d'introduire en outre dans la phase siliconée et/ou cireuse, des ingrédients actifs liposolubles, tels que des filtres U.V., des vitamines liposolubles, des actifs cosmétiques liposolubles.

Les compositions selon l'invention peuvent se présenter sous différentes formes comportant une phase aqueuse et en particulier sous forme d'émulsions huile-dans-eau ou eau-dans-huile ou sous forme de dispersions aqueuses. En particulier la quantité d'eau de la composition représente de 10 à 97,9 % du poids total de la composition et mieux de 30 à 70 %. Selon une forme préférée de réalisation, elles se présentent sous forme d'émulsions huile-dans-eau, qui peut comprendre au moins un tensioactif notamment anionique ou non ionique, en une proportion comprise entre 2 et 30 % en poids par rapport au poids total de la composition,

La composition peut en outre comprendre une matière colorante utilisée de manière usuelle dans le domaine de la cosmétique et du maquillage, telle qu'un colorant hydrosoluble et/ou un pigment.

Les pigments peuvent être présents dans la composition à raison de 0-20% en poids de la composition finale, et de préférence à raison de 1-5%. Ils peuvent être blancs ou colorés, minéraux et/ou organiques, de taille usuelle ou nanométrique. On peut citer, parmi les pigments et nanopigments minéraux, les oxydes de titane, de zirconium ou de cérium. ainsi que les oxydes de zinc, de fer ou de chrome, le bleu ferrique. Parmi les pigments organiques, on peut citer le noir de carbone, et les laques de baryum, strontium, calcium, aluminium.
Parmi les colorants hydrosolubles, on peut citer les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, et leurs mélanges.

On peut également ajouter dans la composition selon l'invention tout additif connu tel que des agents épaississants, par exemple des argiles, des gommes, des silices. les dérivés cellulosiques, un polymère synthétique tel qu'un polymère acrylique ou un polymère associatif de type polyuréthanne; une gomme naturelle telle que la gomme xanthane; des agents d'étalement; des dispersants; des conservateurs: des agents antimousses; des agents mouillants; des filtres UV; des parfums; des charges; des actifs cosmétiques comme les hydratants, les vitamines et leurs dérivés; les matières biologiques et leurs dérivés ; des actifs dermatologiques en vue de conférer à la composition des propriétés de soin et/ou de traitement thérapeutique à application topique.

Bien entendu l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

Cette composition doit bien entendu être apte à se déposer sur un support tel que la peau et/ou les lèvres.

La composition selon l'invention peut se présenter sous forme fluide, gélifiée, semi-solide. pâte souple, voire solide telle que de stick ou bâton. Dans le cas d'une formule non solide, la composition selon l'invention peut présenter une viscosité allant de 0.05 Pa.s à 40 Pa.s (50 cPs à 40000 cPs), et notamment de 0,05 Pa.s à 10 Pa.s, mesurée à 25°C à l'aide d'un appareil Brookfield, mobile 4 LVT. Elle trouve en particulier une application pour la préparation ou en tant que produit de maquillage, notamment en tant que rouge à lèvres, fond de teint, fard à joues ou fard à paupières, ou encore eye-liner. On peut également envisager une application dans le domaine des compositions de soin, des compositions solaires ou autobronzantes, à appliquer sur la peau et/ou les lèvres.

Le démaquillage des compositions selon l'invention se fait aisément avec des démaquillants classiques et/ou des huiles démaquillantes. Il peut également être réalisé à l'aide d'eau chaude savonneuse ou de démaquillants biphasés (huile/eau) usuels.

L'invention est illustrée plus en détail dans les exemples suivants.

### A/ Mesure de l'élongation

L'élongation du film obtenu est mesurée selon la norme ASTM Standards, volume 06.01 D 2370-92 'Standard Test Method for Tensile Properties of Organic Coatings'.

### B/ Mesure de la dureté

La dureté du film est mesurée selon la norme ASTM D-43-66, ou la norme NF-T 30-016 (octobre 1981), à l'aide d'un pendule de Persoz. Le film déposé sur le support doit avoir une épaisseur d'environ 300 microns avant séchage. Après séchage pendant 24 heures, à 30°C et sous une humidité relative de 50%, on obtient un film ayant une épaisseur d'environ 100 microns; on mesure alors sa dureté à 30°C et 50% d'humidité relative.

### C/ Mesure du module de Younq (ou module d'élasticité)

Le module de Young (module d'élasticité) est mesuré selon la norme ASTM Standards, volume 06.01 D 2370-92 'Standard Test Method for Tensile Properties of Organic Coatings'.
Le film déposé sur le support doit avoir une épaisseur d'environ 300 microns avant séchage. Après séchage pendant 7 jours à 21°C et sous une humidité relative de 50%, on obtient un film ayant une épaisseur d'environ 100 microns.
Les échantillons mesurés ont une largeur de 5 mm et une épaisseur de 100 microns. La distance entre les mors est de 25 mm. La vitesse de traction est de 1000 mm par minute.

### Exemple 1

On a préparé des compositions aqueuses comprenant :
- 4,8% de pâte pigmentaire qui comprend 10-16% en poids de glycérine, de manière à obtenir 2% en poids de pigments dans la composition, et
- les composés suivants :

| | |
|---|---|
| composition A | émulsion aqueuse de PDMS (47,5% MA) |
| composition B | dispersion aqueuse de polymère acrylique (42,7% MA) |
| composition C | émulsion aqueuse de PDMS (20,2% MA) + dispersion aqueuse |
| | de polymère acrylique (18,2% MA), selon l'invention. |

On applique les compositions sur les lèvres de 10 personnes qui évaluent de manière sensorielle les critères suivants : brillance, transfert, collant, démaquillage à l'eau froide, démaquillage à l'eau chaude savonneuse (1% de teepol dans l'eau) et démaquillage à l'huile.

On obtient les résultats suivants :

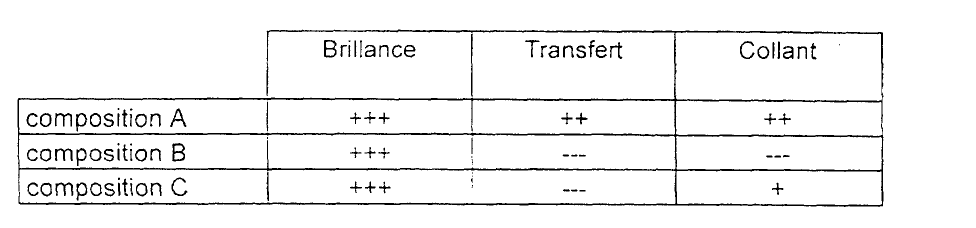

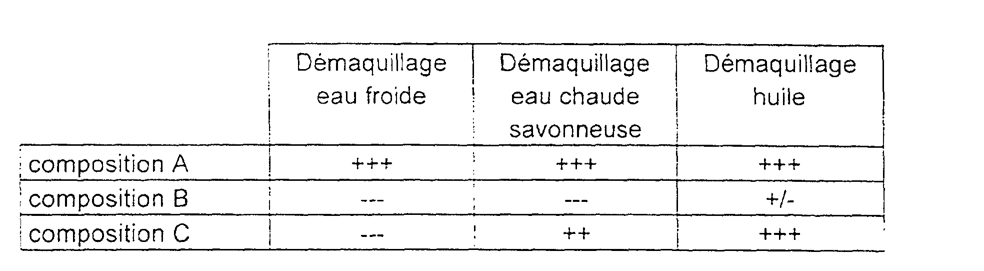

On a donc constaté que la composition C selon l'invention se démaquille aisément à l'aide d'un démaquillant huileux classique, ou d'eau chaude savonneuse.
De plus, on a constaté que la composition C selon l'invention ne transférait pas du tout et était peu collante lorsqu'elle était appliquée sur les lèvres et était brillante.

### Exemple 2

On prépare une composition comprend les constituants suivants :
- dispersion aqueuse de polymère acrylique (45% de matière sèche) 13% MA
- émulsion aqueuse de silicone (50% de matière sèche) 14,5% MA
- dispersion aqueuse de cire polymérique (25% de matière sèche) 7% MA
- pigment 2%
- glycérine 1,25%
- gélifiant 0,9%
- eau qsp 100%

La cire est préalablement dispersée dans l'eau puis l'on ajoute les autres composés. On obtient une composition fluide de rouge à lèvres qui s'applique aisément sur les lèvres, et forme un film brillant, légèrement collant, qui ne transfère pas et résiste à l'eau. Il se démaquille aisément à l'aide d'une huile démaquillante classique.

### Exemple 3

On prépare une composition comprend les constituants suivants
- dispersion aqueuse de polymère acrylique (45% de matière sèche) 18% MA
- émulsion aqueuse de silicone (50% de matière sèche) 20,5% MA
- pigment 2%
- glycérine 1,25%
- gélifiant 1,2%
- eau qsp 100%

On obtient une composition fluide de rouge à lèvres qui s'applique aisément sur les lèvres, et forme un film très brillant, qui ne transfère pas et se démaquille aisément avec des huiles démaquillantes classiques.

## Revendications

1. Composition cosmétique ou dermatologique susceptible d'être appliquée sur la peau et/ou les lèvres, comprenant une quantité suffisante d'un système polymérique qui comprend des particules d'au moins un polymère filmogène en dispersion dans un milieu cosmétiquement ou dermatologiquement acceptable et au moins une émulsion aqueuse d'au moins un composé siliconé, la quantité de matière sèche de polymère filmogène allant de 2 à 60 % du poids total de la composition et la quantité de composé siliconé allant de 0,1 à 30 % en poids de matière sèche de silicone, par rapport au poids total de la composition.

2. Composition selon la revendication 1, caractérisée en ce que le polymère filmogène est présent dans la composition sous forme de dispersion aqueuse de particules.

3. Composition selon la revendication 2, dans laquelle la taille des particules de polymère en dispersion aqueuse est comprise entre 10-500 nm, de préférence entre 20 et 150 nm.

4. Composition selon l'une des revendications précédentes, dans laquelle le polymère filmogène est choisi parmi les polymères synthétiques, de type polycondensat ou de type radicalaire, les polymères d'origine naturelle, et leurs mélanges.

5. Composition selon l'une des revendications précédentes, dans laquelle le polymère filmogène est choisi parmi les polyuréthannes anioniques, cationiques, non ioniques ou amphotères; les polyuréthannes-acryliques; les polyuréthannes-polyvinylpirrolidones; les polyester-polyuréthannes; les polyéther-polyuréthannes; les polyurées; les polyurée/polyuréthannes; les polyesters; les polyesters amides; les polyesters à chaîne grasse; les polyamides; les résines époxyesters; les polymères ou copolymères acryliques et/ou vinyliques; les copolymères acryliques/silicones; les copolymères nitrocellulose/acryliques; la résine shellac, la gomme de sandaraque, les dammars, les élémis, les copals, les dérivés cellulosiques; les polymères hybrides; leurs mélanges.

6. Composition selon l'une des revendications précédentes, dans laquelle le système polymérique est tel qu'il forme sur la peau et/ou les lèvres un film sans transfert et/ou résistant à l'eau et/ou brillant et/ou ne tachant pas et/ou ne migrant pas et/ou de bonne tenue.

7. Composition selon l'une des revendications précédentes, dans laquelle le système polymérique est apte à former un film vérifiant au moins une des conditions suivantes :
. un module de Young inférieur à environ 200 MPa, de préférence inférieur à environ 100 MPa, et préférentiellement inférieur à 80 MPa, et/ou
. une élongation supérieure à environ 200%, et, de manière préférentielle, supérieure à 300%, et/ou
. une dureté inférieure à 110, de préférence inférieure à 70, plus préférentiellement inférieure à 55.

8. Composition selon l'une des revendications précédentes, dans laquelle la quantité de matières sèches de polymère filmogène va de 5 à 60 % du poids total de la composition.

9. Composition selon l'une des revendications précédentes, dans laquelle l'émulsion aqueuse d'au moins un composé siliconé a une taille des gouttelettes inférieure à 1 µm, notamment une taille de gouttelettes de l'ordre de 10 à 80 nm.

10. Composition selon l'une des revendications précédentes, dans laquelle le composé siliconé présent dans l'émulsion est choisi parmi les polyorganosiloxanes, notamment les polyalkylsiloxanes, les polyarylsiloxanes, les polyalkylarylsiloxanes, les copolymères polyéthersiloxanes, organomodifiés ou non, les gommes et résines de silicones, les polysiloxanes modifiés par des groupements organofonctionnels, les silicones volatils, ainsi que leurs mélanges.

11. Composition selon l'une des revendications précédentes, dans laquelle le composé siliconé est choisi parmi les polydiméthylsiloxanes linéaires à groupements terminaux triméthylsilyle; les polydiméthylsiloxanes linéaires à groupements terminaux diméthylsilanol; les polydiméthylméthylphénylsiloxanes; les polydiméthyldiphénylsiloxanes.

12. Composition selon l'une des revendications précédentes, dans laquelle le composé siliconé est présent dans la composition à raison de 5 % à 30% en poids de matière sèche de silicone.

13. Composition selon l'une des revendications précédentes, comprenant, en outre, une dispersion aqueuse de cire, de préférence une microdispersion aqueuse de cire.

14. Composition selon la revendication 13, dans laquelle la cire est choisie parmi la cire d'abeilles, la cire de lanoline, les cires d'insectes de Chine, la cire de riz, la cire de Carnauba, la cire de Candellila, la cire d'Ouricurry, la cire d'Alfa, la cire de fibres de liège, la cire de canne à sucre, la cire du Japon, la cire de sumac, la cire de montan, les cires microcristallines, les paraffines, l'ozokérite, les cires de polyéthylène, les cires obtenues par la synthèse de Fisher-Tropsch, les copolymères cireux ainsi que leurs esters, les cires obtenues par hydrogénation catalytique d'huiles animales ou végétales ayant des chaînes grasses, linéaires ou ramifiées, en C8-C32, les cires de silicone, les cires fluorées.

15. Composition selon l'une des revendications précédentes, se présentant sous la forme d'une composition de maquillage telle que rouge à lèvres, fond de teint, fard à joues, fard à paupières, eye-liner; d'une composition de soin; d'une composition solaire ou autobronzante.

16. Composition selon l'une des revendications précédentes, comprenant, en outre, une quantité suffisante de tensio-actif pour obtenir une microdispersion de cire et/ou une émulsion de silicone.

17. Composition selon l'une des revendications précédentes, comprenant de 10 à 97,9 % d'eau en poids par rapport au poids total de la composition, et mieux de 30 à 70 %.

18. Composition selon l'une des revendications précédentes, comprenant, en outre, une matière colorante.

19. Composition de maquillage ou de soin sans transfert, comprenant une quantité efficace d'un système polymérique qui comprend des particules d'au moins un polymère filmogène en dispersion dans un milieu cosmétiquement ou dermatologiquement acceptable et au moins une émulsion aqueuse d'au moins un composé siliconé, la quantité de matière sèche de polymère filmogène allant de 2 à 60 % du poids total de la composition et la quantité de composé siliconé allant de 0,1 à 30% en poids de matière sèche de silicone, par rapport au poids total de la composition.

20. Composition de maquillage des lèvres du visage et/ou de la peau des êtres humains, comprenant une quantité efficace d'un système polymérique qui comprend des particules d'au moins un polymère filmogène en dispersion dans un milieu cosmétiquement ou dermatologiquement acceptable et au moins une émulsion aqueuse d'au moins un composé siliconé, la quantité de matière sèche de polymère filmogène allant de 2 à 60 % du poids total de la composition et la quantité de composé siliconé allant de 0,1 à 30 % en poids de matière sèche de silicone, par rapport au poids total de la composition.

21. Utilisation de l'association d'un système polymérique qui comprend en quantité efficace des particules d'au moins un polymère filmogène en dispersion dans un milieu cosmétiquement acceptable, et d'une émulsion aqueuse d'au moins un composé siliconé, dans une composition cosmétique susceptible d'être appliquée sur la peau et/ou les lèvres, afin d'obtenir sur la peau et/ou les lèvres, un film cohésif de très bonne tenue et/ou qui ne transfère pas et/ou qui ne migre pas et/ou qui ne tache pas et/ou qui ne colle pas.

22. Utilisation de l'association d'un système polymérique en quantité efficace qui comprend des particules d'au moins un polymère filmogène en dispersion dans un milieu dermatologiquement acceptable, et d'une émulsion aqueuse d'au moins un composé siliconé, pour la fabrication d'une composition destinée à traiter thérapeutiquement la peau et/ou les lèvres et former un film cohésif sur la peau et/ou les lèvres, suivant leurs mouvements.

## Claims

1. Cosmetic or dermatological composition which may be applied to the skin and/or the lips, comprising a sufficient amount of a polymer system which comprises particles of at least one film-forming polymer dispersed in a cosmetically or dermatologically acceptable medium and at least one aqueous emulsion of at least one silicone compound, the amount of film-forming polymer solids ranging from 2% to 60% of the total weight of the composition and the amount of silicone compound ranging from 0.1% to 30% by weight of silicone solids relative to the total weight of the composition.

2. Composition according to Claim 1, characterized in that the film-forming polymer is present in the composition in the form of an aqueous dispersion of particles.

3. Composition according to Claim 2, in which the size of the polymer particles in aqueous dispersion is between 10-500 nm and preferably between 20 and 150 nm.

4. Composition according to one of the preceding claims, in which the film-forming polymer is chosen from synthetic polymers, of polycondensate type or of radical type, and polymers of natural origin, and mixtures thereof.

5. Composition according to one of the preceding claims, in which the film-forming polymer is chosen from anionic, cationic, nonionic and amphoteric polyurethanes; polyurethane-acrylics; polyurethane-polyvinylpyrrolidones; polyester-polyurethanes; polyether-polyurethanes; polyureas; polyurea-polyurethanes; polyesters; polyesteramides; fatty-chain polyesters; polyamides; epoxy ester resins; acrylic and/or vinyl polymers or copolymers; acrylic/silicone copolymers; nitrocellulose/acrylic copolymers; shellac resin, sandarac gum, dammar resins, elemi gums, copal resins, cellulose derivatives; hybrid polymers; mixtures thereof.

6. Composition according to one of the preceding claims, in which the polymer system is such that it forms on the skin and/or the lips a transfer-resistant and/or water-resistant and/or glossy and/or mark-resistant and/or migration-resistant film and/or a film with good staying power.

7. Composition according to one of the preceding claims, in which the polymer system is capable of forming a film which satisfies at least one of the following conditions:
a Young's modulus of less than about 200 MPa, preferably less than about 100 MPa and preferentially less than 80 MPa, and/or
· an elongation of greater than about 200% and preferably greater than 300%, and/or
· a hardness of less than 110, preferably less than 70 and more preferably less than 55.

8. Composition according to one of the preceding claims, in which the amount of film-forming polymer solids ranges from 5% to 60% of the total weight of the composition.

9. Composition according to one of the preceding claims, in which the aqueous emulsion of at least one silicone compound has a droplet size of less than 1 *µ*m, in particular a droplet size of about from 10 to 80 nm.

10. Composition according to one of the preceding claims, in which the silicone compound present in the emulsion is chosen from polyorganosiloxanes, in particular polyalkylsiloxanes, polyarylsiloxanes, poly-alkylarylsiloxanes, polyethersiloxane copolymers, which may or may not be organomodified, silicone gums and resins, polysiloxanes modified with organofunctional groups, and volatile silicones, as well as mixtures thereof.

11. Composition according to one of the preceding claims, in which the silicone compound is chosen from linear polydimethylsiloxanes containing trimethylsilyl end groups; linear polydimethylsiloxanes containing dimethylsilanol end groups; polydimethylmethylphenyl-siloxanes; polydimethyldiphenylsiloxanes.

12. Composition according to one of the preceding claims, in which the silicone compound is present in the composition in a proportion of from 5% to 30% by weight of silicone solids.

13. Composition according to one of the preceding claims, also comprising an aqueous dispersion of wax, preferably an aqueous microdispersion of wax.

14. Composition according to Claim 13, in which the wax is chosen from beeswax, lanolin wax, Chinese insect waxes, rice wax, carnauba wax, candelilla wax, ouricurry wax, alfalfa wax, cork fibre wax, sugarcane wax, Japan wax, sumac wax, montan wax, microcrystalline waxes, paraffins, ozokerite, polyethylene waxes, the waxes obtained by Fisher-Tropsch synthesis, waxy copolymers and esters thereof, the waxes obtained by catalytic hydrogenation of animal or plant oils containing linear or branched C8-C32 fatty chains, silicone waxes and fluorowaxes.

15. Composition according to one of the preceding claims, which is in the form of a make-up composition such as a lipstick, a foundation, a face powder, an eyeshadow, an eyeliner; a care composition; an antisun composition or a self-tanning composition.

16. Composition according to one of the preceding claims, also comprising an amount of surfactant which is sufficient to obtain a wax microdispersion and/or a silicone emulsion.

17. Composition according to one of the preceding claims, comprising from 10% to 97.9% by weight of water relative to the total weight of the composition, and better still from 30% to 70%.

18. Composition according to one of the preceding claims, also comprising a colorant.

19. Transfer-resistant make-up or care composition, comprising an effective amount of a polymer system which comprises particles of at least one film-forming polymer dispersed in a cosmetically or dermatologically acceptable medium and at least one aqueous emulsion of at least one silicone compound, the amount of film-forming polymer solids ranging from 2% to 60% of the total weight of the composition and the amount of silicone compound ranging from 0.1% to 30% by weight of silicone solids relative to the total weight of the composition.

20. Make-up composition for the lips of the face and/or for the skin of human beings, comprising an effective amount of a polymer system which comprises particles of at least one film-forming polymer dispersed in a cosmetically or dermatologically acceptable medium and at least one aqueous emulsion of at least one silicone compound, the amount of film-forming polymer solids ranging from 2% to 60% of the total weight of the composition and the amount of silicone compound ranging from 0.1% to 30% by weight of silicone solids relative to the total weight of the composition.

21. Use of a combination of a polymer system which comprises an effective amount of particles of at least one film-forming polymer dispersed in a cosmetically acceptable medium, and of an aqueous emulsion of at least one silicone compound, in a cosmetic composition which may be applied to the skin and/or the lips in order to give the skin and/or the lips a cohesive film with very good staying power and/or a film which does not transfer and/or which does not migrate and/or which does not leave marks and/or which is not sticky.

22. Use of a combination of a polymer system in an effective amount, which comprises particles of at least one film-forming polymer dispersed in a dermatologically acceptable medium, and of an aqueous emulsion of at least one silicone compound, for the manufacture of a composition for therapeutically treating the skin and/or the lips and for forming a cohesive film on the skin and/or the lips, which follows their movements.

## Patentansprüche

1. Kosmetische oder dermatologische Zusammensetzung, die auf die Haut und/oder die Lippen aufgetragen werden kann und die in einer wirksamen Menge ein Polymersystem, das Partikel mindestens eines filmbildenden Polymers in Dispersion in einem kosmetisch oder dermatologisch akzeptablen Medium enthält, und mindestens eine wäßrige Emulsion mindestens einer Siliconverbindung enthält, wobei der Trockensubstanzgehalt des filmbildenden Polymers im Bereich von 2 bis 60 % des Gesamtgewichts der Zusammensetzung und die Menge der Siliconverbindung im Bereich von 0,1 bis 30 Gew.-% Trockensubstanz Silicon, bezogen auf das Gesamtgewicht der Zusammensetzung, liegt.

2. Zusammensetzung nach Anspruch 1, dadurch gekennzeichnet, daß das filmbildende Polymer in der Zusammensetzung in Form einer wäßrigen Dispersion von Partikeln vorliegt.

3. Zusammensetzung nach Anspruch 2, wobei die Polymerpartikel in wäßriger Dispersion eine Größe im Bereich von 10 bis 500 nm und vorzugsweise im Bereich von 20 bis 150 nm aufweisen.

4. Zusammensetzung nach einem der vorhergehenden Ansprüche, wobei das filmbildende Polymer unter den synthetischen Polymeren vom Polykondensat-Typ oder vom Radikal-Typ, den Polymeren natürlichen Ursprungs und deren Gemischen ausgewählt ist.

5. Zusammensetzung nach einem der vorhergehenden Ansprüche, wobei das filmbildende Polymer ausgewählt ist unter den anionischen, kationischen, nichtionischen oder amphoteren Polyurethanen; Polyurethan-Acryl-Polymeren; Polyurethan-Polyvinylpyrrolidonen; Polyester-Polyurethanen; Polyether-Polyurethanen; Polyharnstoffen; Polyharnstoff/Polyurethanen; Polyestern; Polyesteramiden; Polyestern mit Fettkette; Polyamiden; Epoxyesterharzen;, Acryl- und/oder Vinylpolymeren oder -copolymeren; Acryl/Siliconcopolymeren; Nitrocellulose/Acrylcopolymeren; Schellak, Sandarak, Dammarharzen, Elemi, Kopalen, Cellulosederivaten; Hybridpolymeren und deren Gemischen.

6. Zusammensetzung nach einem der vorhergehenden Ansprüche, wobei das Polymersystem so vorliegt, daß es auf der Haut und/oder den Lippen einen Film ausbildet, der sich nicht überträgt und/oder gegenüber Wasser beständig ist und/oder glänzt und/oder keine Flecken verursacht und/oder keine Migration zeigt und/oder gut haftet.

7. Zusammensetzung nach einem der vorhergehenden Ansprüche, wobei durch das Polymersystem ein Film erhalten werden kann, der mindestens eine der folgenden Bedingungen erfüllt:
- ein Young-Modul unter etwa 200 MPa, vorzugsweise unter etwa 100 MPa und noch bevorzugter unter 80 MPa, und/oder
- eine Dehnung über etwa 200 % und vorzugsweise über 300 %, und/oder
- eine Härte unter 110, vorzugsweise unter 70 und noch bevorzugter unter 55.

8. Zusammensetzung nach einem der vorhergehenden Ansprüche, wobei der Trockensubstanzgehalt des filmbildenden Polymers im Bereich von 5 bis 60 % des Gesamtgewichts der Zusammensetzung liegt.

9. Zusammensetzung nach einem der vorhergehenden Ansprüche, wobei die Größe der Tröpfchen in der wäßrigen Emulsion mindestens einer Siliconverbindung unter 1 µm und insbesondere in der Größenordnung von 10 bis 80 nm liegt.

10. Zusammensetzung nach einem der vorhergehenden Ansprüche, wobei die in der Emulsion vorliegende Siliconverbindung unter den Polyorganosiloxanen, insbesondere den Polyalkylsiloxanen, Polyarylsiloxanen, Polyalkylarylsiloxanen, gegebenenfalls organomodifizierten Copolymeren von Polyethersiloxanen, Silicongummis und Siliconharzen, mit organofunktionellen Gruppen modifizierten Polysiloxanen, flüchtigen Siliconen sowie deren Gemischen ausgewählt ist.

11. Zusammensetzung nach einem der vorhergehenden Ansprüche, wobei die Siliconverbindung unter den geradkettigen Polydimethylsiloxanen mit Trimethylsilylendgruppen, geradkettigen Polydimethylsiloxanen mit Trimethylsilanolendgruppen, Polydimethylmethylphenylsiloxanen und Polydimethyldiphenylsiloxanen ausgewählt ist.

12. Zusammensetzung nach einem der vorhergehenden Ansprüche, wobei die Siliconverbindung in der Zusammensetzung in einem Anteil von 5 bis 30 Gew.-% Trokkensubstanz Silicon vorliegt.

13. Zusammensetzung nach einem der vorhergehenden Ansprüche, wobei sie ferner eine wäßrige Dispersion eines Wachses und vorzugsweise eine wäßrige Mikrodispersion eines Wachses enthält.

14. Zusammensetzung nach Anspruch 13, wobei das Wachs ausgewählt ist unter: Bienenwachs, Lanolinwachs, Chinawachsen, Reiswachs, Camaubawachs, Candellilawachs, Ouricurywachs, Alfawachs, Korkfaserwachs, Zuckerrohrwachs, Japanwachs, Sumachwachs, Montanwachs, mikrokristallinen Wachsen, Paraffinen, Ozokerit, Polyethylenwachsen, durch Fischer-Tropsch-Synthese hergestellten Wachsen, wachsigen Copolymeren sowie deren Estern, Wachsen, die durch katalytische Hydrierung von tierischen oder pflanzlichen Ölen mit geradkettigen oder verzweigten C₈₋₃₂-Fettketten hergestellt sind, Siliconwachsen und fluorierten Wachsen.

15. Zusammensetzung nach einem der vorhergehenden Ansprüche, die in Form eines Produkts zum Schminken, beispielsweise als Lippenstift, Make-up, Wangenrouge, Lidschatten oder Eyeliner, einer Zusammensetzung zur Pflege, einer Zusammensetzung zum Sonnenschutz oder einer Zusammensetzung zur Selbstbräunung vorliegt.

16. Zusammensetzung nach einem der vorhergehenden Ansprüche, die ferner einen grenzflächenaktiven Stoff in einer Menge enthält, die ausreichend ist, um eine Wachsmikrodispersion und/oder eine Siliconemulsion zu erhalten.

17. Zusammensetzung nach einem der vorhergehenden Ansprüche, die 10 bis 97,9 Gew.-% Wasser, bezogen auf das Gesamtgewicht der Zusammensetzung, und besser 30 bis 70 % Wasser enthält.

18. Zusammensetzung nach einem der vorhergehenden Ansprüche, die ferner ein Färbemittel enthält.

19. Zusammensetzung zum Schminken oder zur Pflege ohne Transfer, die in einer wirksamen Menge ein Polymersystem, das Partikel mindestens eines filmbildenden Polymers in Dispersion in einem kosmetisch oder dermatologisch akzeptablen Medium enthält, und mindestens eine wäßrige Emulsion mindestens einer Siliconverbindung enthält, wobei der Trockensubstanzgehalt des filmbildenden Polymers im Bereich von 2 bis 60 % des Gesamtgewichts der Zusammensetzung und die Menge der Siliconverbindung im Bereich von 0,1 bis 30 Gew.-% Trockensubstanz Silicon, bezogen auf das Gesamtgewicht der Zusammensetzung, liegt.

20. Zusammensetzung zum Schminken der menschlichen Lippen und/oder der Haut, die in einer wirksamen Menge ein Polymersystem, das Partikel mindestens eines filmbildenden Polymers in Dispersion in einem kosmetisch oder dermatologisch akzeptablen Medium enthält, und mindestens eine wäßrige Emulsion mindestens einer Siliconverbindung enthält, wobei der Trockensubstanzgehalt des filmbildenden Polymers im Bereich von 2 bis 60 % des Gesamtgewichts der Zusammensetzung und die Menge der Siliconverbindung im Bereich von 0,1 bis 360 Gew.-% Trockensubstanz Silicon, bezogen auf das Gesamtgewicht der Zusammensetzung, liegt.

21. Verwendung eines Polymersystems, das in einer wirksamen Menge Partikel mindestens eines filmbildenden Polymers in Dispersion in einem kosmetisch akzeptablen Medium enthält, in Kombination mit einer wäßrigen Emulsion mindestens einer Siliconverbindung in einer kosmetischen Zusammensetzung, die auf die Haut und/oder die Lippen aufgetragen werden kann, um auf der Haut und/oder den Lippen einen kohäsiven Film zu bilden, der sehr gut haftet und/oder sich nicht überträgt und/oder keine Migration zeigt und/oder keine Flecken verursacht und/oder nicht klebrig ist.

22. Verwendung einer wirksamen Menge eines Polymersystems, das Partikel mindestens eines filmbildenden Polymers in Dispersion in einem dermatologisch akzeptablen Medium enthält, in Kombination mit einer wäßrigen Emulsion mindestens einer Siliconverbindung zur Herstellung einer Zusammensetzung, die zur therapeutischen Behandlung der Haut und/oder der Lippen vorgesehen ist und auf der Haut ünd/oder den Lippen einen kohäsiven Film bilden soll, der ihren Bewegungen folgt.
